# FASCICULE DE BREVET EUROPEEN

(11) **EP 3 930 830 B1**
(45) Date de publication et mention de la délivrance du brevet: **07.06.2023**
(21) Numéro de dépôt: 20707612.6
(22) Date de dépôt: 27.02.2020
(51) Int. Cl.: A61N 5/02, A61N 5/04, A44C 5/00

(54) **DISPOSITIF COMPRENANT UNE SOURCE DE CHALEUR**
VORRICHTUNG MIT EINER WÄRMEQUELLE
DEVICE COMPRISING A HEAT SOURCE

(30) Priorité: 01.03.2019 FR 1902167
(43) Date de publication de la demande: 05.01.2022
(73) Titulaire: Remedee Labs, 38240 Meylan (FR)
(72) Inventeur: LAMBERT, Aurélien, 38600 FONTAINE (FR); FOERSTER, Michael, 38700 CORENC (FR)
(74) Mandataire: LLR
(86) Numéro de dépôt international: PCT/EP2020/055078
(87) Numéro de publication internationale: WO 2020/178111

(56) Documents cités:
- CN-A- 106 418 924
- US-A1- 2015 119 963

## Description

L'invention concerne les dispositifs embarquant de l'électronique. Elle concerne en particulier la gestion de la dissipation thermique au sein d'un dispositif visant à traiter la douleur par émission d'ondes millimétriques.

On connaît déjà dans l'état la technique, notamment d'après le document US2017/0303433 (Delano), un bracelet connecté comprenant un système de dissipation thermique incluant deux couches conductrices de chaleur entourant une couche de matériau à changement de phase. Cet empilement permet de dissiper une partie de la chaleur émise par l'électronique du bracelet, mais pas suffisamment en cas de forte émission de chaleur.

Le document US 2015/119963 divulgue un bracelet comportant un boîtier comprenant une source de chaleur et un brin plus souple que le boîtier. La source de chaleur est un matériau à changement de phase.

Or, pour le confort du sujet humain portant le bracelet, il est nécessaire de maintenir la température de la peau sous une valeur prédéterminée.

L'invention a notamment pour but d'améliorer la gestion de la dissipation de chaleur au sein d'un dispositif connecté.

A cet effet l'invention a pour objet un dispositif comportant :
- au moins un boitier comprenant au moins une source de chaleur, et
- au moins un brin formant une partie plus souple que le boitier,
le brin comprenant :
- deux couches de matériau conductrices de chaleur, et
- une couche de matériau à changement de phase située entre les deux couches de matériau conductrices de chaleur.

Ainsi, la gestion de la dissipation thermique tire parti de la longueur du brin, ou anse ou arceau, du dispositif pour augmenter au maximum la surface de dissipation de la chaleur émise par la source, chaleur se dissipant tout le long d'une des couches de matériau conductrice, puis au sein de la couche de matériau à changement de phase, puis, pour ce qu'il en reste, tout le long de l'autre couche conductrice. La dissipation n'est donc pas confinée au boitier où est émise la chaleur, mais s'étend au sein d'une partie souple à l'extérieur du boitier. De cette manière, la chaleur est dissipée au maximum et la température du dispositif au contact de la peau, au niveau de la source de chaleur, est réduite.

Avantageusement, le dispositif est un bracelet.

Par « bracelet », on entend tout objet en forme d'anneau qui peut être porté sur soi, par exemple autour du poignet, du bras ou d'une cheville. L'invention permet de réduire la chaleur du bracelet au contact du de la peau, par exemple au contact du poignet.

De préférence, le boitier est rigide.

Ainsi, l'invention permet d'assurer la gestion de la dissipation thermique du dispositif alors que la source de chaleur n'est pas aisément déformable ou déplaçable.

Avantageusement, une valeur de conductivité thermique d'au moins l'une des couches conductrices de chaleur est supérieure ou égale à 350 watts par mètre-kelvin, de préférence supérieure ou égale à 1300 watts par mètre-kelvin.

La valeur de 350 watts par mètre-kelvin correspond environ à la conductivité thermique du cuivre, et des essais ont démontré la suffisance de feuilles de cuivre comme couches conductrices dans certaines conditions. Il est toutefois à noter que plus la conductivité est élevée, plus il est possible de diminuer l'épaisseur de la couche pour un même niveau de dissipation. Les couches dont la conductivité est supérieure ou égale à 1300 watts par mètre-kelvin peuvent ainsi être très fines et donc permettre de gagner de la place au sein du dispositif.

De préférence, une valeur d'épaisseur d'au moins l'une des couches conductrices de chaleur est située entre 0.01 et 1 millimètre.

Ainsi, plus la couche est conductrice, plus elle peut être fine. Le dispositif peut donc lui aussi être fin, ce qui peut être confortable, esthétiquement intéressant ou plus économique à produire.

Avantageusement, une valeur d'enthalpie de changement de phase de la couche de matériau à changement de phase est d'environ 100 joules par gramme.

De manière générale, plus l'enthalpie est importante, plus le matériau a changement de phase va pouvoir absorber de la chaleur, ce qui est intéressant en particulier dans le cadre d'un régime transitoire où la source de chaleur fournit une forte puissance thermique sur une courte durée.

De préférence, les deux couches de matériau conductrices de chaleur sont collées à deux surfaces opposées de la couche de matériau à changement de phase.

Ainsi, les trois couches forment un empilement compact, étant séparées éventuellement et uniquement par deux enduis adhésifs situés entre une première couche conductrice et la couche de matériau à changement de phase d'une part, entre cette dernière et la deuxième couche conductrice d'autre part. La chaleur est donc transmise directement d'une couche de l'empilement à l'autre, ce qui améliore sa dissipation tout en diminuant l'espace pris par l'empilement formé par les trois couches.

Avantageusement, au moins l'une des trois couches s'étend également dans le boitier.

Ainsi, la ou chaque couche est au plus près de la source de chaleur, dans le boitier qui peut notamment être en contact avec la surface inférieure ou supérieure du poignet d'un sujet humain. La ou chaque couche s'étend également à l'extérieur du boitier, au sein du brin formant partie souple et fermant le dispositif. La chaleur part ainsi de la partie rigide, le boitier où est situé la source de chaleur, et est dissipée en s'étendant le long du dispositif.

De préférence, le dispositif comprend en outre une nappe de connexion électronique s'étendant depuis le boitier et dans le brin, au moins l'une des couches de matériau conductrices de chaleur étant fixée à la nappe dans le brin.

Ainsi, la nappe de connexion permet de connecter l'électronique du boitier avec un autre élément du dispositif. Cet autre élément peut correspondre à des éléments électroniques situés en dehors du boitier, voire dans un autre boitier, par exemple situé en face du premier. Ainsi, la couche tire parti de la présence de cette nappe pour s'étendre dans le brin. Elle utilise donc la présence d'un élément qui est déjà là à d'autres fins - connecter l'électronique - pour s'étendre dans le brin, ce qui permet de ne pas complexifier la structure du dispositif.

Avantageusement, au moins l'une des couches de matériau conductrices de chaleur comprend une feuille de graphite pyrolitique présentant une valeur d'épaisseur située entre 10 et 100 micromètres.

En effet, cette feuille de graphite a un fort pouvoir conducteur de chaleur, environ cinq fois plus important que celle du cuivre, tout en étant fine. L'épaisseur finale dépend de l'agencement du dispositif souhaité.

De préférence, la couche de matériau à changement de phase présente une valeur d'épaisseur située entre 1 et 5 millimètres.

Ainsi, cette épaisseur dépend de l'enthalpie de la couche - plus cette dernière est élevée, plus l'épaisseur peut être faible - et de l'agencement du dispositif.

De manière générale, pour les trois couches de matériau, l'objectif est de trouver un compromis entre une épaisseur la plus faible possible et une dissipation, et donc une enthalpie ou une valeur de conductivité, la plus élevée possible.

Avantageusement, les trois couches étant trois premières couches, le dispositif comprend en outre une quatrième couche de matériau isolante thermique, située entre les trois couches d'une part et une extrémité du boitier d'autre part.

Ainsi, cette couche isolante permet d'éviter l'élévation de température sur le côté du boitier opposé à la source de chaleur. En particulier, ce côté est amené à être en contact avec l'air ambiant, dont la température peut déjà être élevée et qu'il faut donc séparer de la source de chaleur et du système de dissipation de la chaleur formé par les trois couches.

De préférence, une valeur de conductivité de la couche de matériau isolante thermique est située entre 0.018 et 0.026 watts par mètre-kelvin, cette couche comportant de préférence une feuille thermo-isolante comprenant un aérogel de silice et des nanofibres, cette couche présentant de préférence une valeur d'épaisseur située entre 0.1 et 1 millimètres.

On trouve en particulier des matériaux adéquats de 500 micromètres d'épaisseur permettant d'isoler suffisamment thermiquement cette extrémité du boitier.

Avantageusement, le dispositif comprend en outre un pad thermique situé entre la source de chaleur d'une part et les couches de matériau d'autre part, le pad comprenant un matériau présentant de préférence une valeur de conductivité thermique supérieure ou égale à 12.0 watts par mètre-kelvin et une valeur d'épaisseur située entre 0.5 et 3 millimètres.

Ainsi, le pad thermique permet de collecter la chaleur émise par la source de chaleur afin de la transférer à la première couche conductrice. La source de chaleur peut être multiple - un circuit imprimé va comprendre par exemple plusieurs éléments électroniques sources de chaleur - ou encore située à différents niveaux, par exemple à différentes hauteurs sur le circuit imprimé. Le pad thermique, grâce à ses logements formés lors de son assemblage aux sources de chaleurs et adaptés aux niveaux des différentes sources de chaleur, permet à la fois de maintenir la ou les sources de chaleur en position et de transférer efficacement la chaleur émise aux couches de matériau adjacentes.

De préférence, la source de chaleur est au moins un composant de circuit imprimé.

En effet, la circulation et le stockage de charges électriques engendre de la chaleur dans des composants du circuit que sont par exemple des résistances ou des composants plus complexes formant des processeurs ou par exemple des ASIC (Application-Specific Integrated Circuit). Le circuit imprimé peut servir à contrôler ou à alimenter un dispositif électronique quelconque du dispositif, tel qu'un organe de connexion à distance, un écran, un terminal de communication, une interface quelconque, ou encore un organe contrôlé à distance ou préprogrammé réalisant une tâche sur le dispositif, tel qu'un module d'envoi d'ondes.

Avantageusement, la source de chaleur est un module d'émission d'ondes apte à émettre des ondes présentant une valeur de fréquence située entre 3 et 120 gigahertz et une valeur de densité surfacique de puissance supérieure ou égale à 0.5 milliwatts par centimètre carré.

Ainsi, le dispositif sert à envoyer des ondes millimétriques au niveau du d'une peau d'un sujet humain, tel qu'un poignet, une cheville si le dispositif est un bracelet. L'électronique permettant l'envoi de ces ondes engendre une quantité de chaleur importante qu'il faut dissiper. En particulier, il peut être nécessaire, aussi bien pour des raisons de confort que de réglementation, de limiter la température du dispositif au contact de la peau à 43°C, en particulier si la durée du contact avec la source de chaleur est supérieure à dix minutes. Ainsi, dans un mode de mise en oeuvre d'un dispositif connecté comprenant un tel module, la puissance thermique à dissiper est d'environ 1.25 watts pendant trente minutes de fonctionnement. C'est notamment en vue dissiper cette puissance pendant cette durée qu'est conçu le dispositif selon l'invention, les différentes couches faisant partie du système de gestion de la dissipation du dispositif.

De préférence, le dispositif tel que décrit ci-avant est prévu pour utilisation dans le traitement de la douleur.

En effet, on sait que la thérapie par émission d'ondes dites « millimétriques » (c'est-à-dire dont la fréquence est inférieure à 300 gigaherz) provoque la réduction de douleurs (voir la publication Usichenko TI, Edinge H, Gizkho W, Lehmann C, Wend M, Feyerherd F : « Low-intensity electromagnetic millimeter waves for pain therapy. Evid Based Complement Alternat Med »). Il a ainsi été démontré que l'exposition d'une zone du corps humain a des ondes électromagnétiques millimétriques permettait la libération d'opioïdes endogènes (voir la publication Rojavin MA, Ziskin MC : « Electromagnetic Millimeter waves increase the duration of anaesthesia caused by ketamine and chloral hydrate in mice Int J Radiat Biol »), générant au niveau du cerveau la synthèse d'enképhaline, un peptide naturel impliqué dans la tolérance à la douleur. Cette technique permet donc d'éviter les inconvénients du traitement par antalgiques - effets secondaires indésirables - ou par neurostimulation - inconfort du patient - et son fonctionnement est compris. L'envoi d'ondes millimétriques présentant une densité surfacique de puissance de 0.5 milliwatts par centimètre carré au minimum (et plutôt bien au-delà), et cela sur une surface réduite du poignet humain, nécessite une électronique engendrant nécessairement de la chaleur, pouvant correspondre, comme indiqué plus haut, à près de 1.25 watts de puissance thermique émise pendant trente minutes. En particulier, dans ce type de dispositif, la conversion du courant continu d'une batterie en rayonnement électromagnétique présente un faible rendement, engendrant les pertes thermiques évoquées. De plus, ce rendement est meilleur à faible température. Il est donc important, aussi bien pour l'autonomie de la batterie que pour respecter les limites d'échauffement du patient, de gérer au mieux la dissipation thermique. Cela est donc réalisé au moyen des caractéristiques du système de gestion de la dissipation du dispositif décrit plus haut.

Avantageusement, le boitier comprend en outre un capteur de température apte à stopper une émission de chaleur de la source de chaleur si une valeur de température du dispositif dépasse un seuil prédéterminé, par exemple si une valeur de température de la source de chaleur dépasse 55°C.

Ainsi, cette sécurité matérielle, indépendante de tout logiciel qui serait mis en oeuvre dans le dispositif, permet de couper toute puissance si cette température de 55°C est atteinte. Bien entendu, une autre température pourrait être prédéterminée, mais comme toute sécurité matérielle, elle a vocation à être figée dès la conception du dispositif et à ne pas pouvoir être modifiée, en tout cas aisément, par un utilisateur. Il s'agit ainsi d'une sécurité de dernier recours si les sécurités logicielles, qui auraient dû d'ores et déjà stopper la source de chaleur, n'ont pas fonctionné comme souhaité initialement.

De préférence, le dispositif inclue en outre un support électronique comprenant sous forme enregistrée un programme d'ordinateur, le programme comportant des instructions de code aptes à activer et/ou désactiver une émission de chaleur de la source de chaleur en fonction d'une valeur de température du dispositif, par exemple apte à désactiver l'émission si la valeur est supérieure à 43°C et à activer l'émission si la valeur est inférieure à 41°C.

Ainsi, il s'agit d'une ou plusieurs sécurités logicielles. La première consiste mettre en pause le système à l'origine de la source de chaleur si la température de cette dernière au niveau de la peau du sujet dépasse 43°C. Une fois qu'elle est redescendue sous 41°C, le système peut être redémarré. Une deuxième sécurité logicielle peut stopper toute puissance si la température dépasse 50°C. Cette deuxième sécurité peut être prévue de sorte que, une fois déclenchée, le dispositif ne peut être réactivé que par une personne prédéterminée, par exemple une personne d'un service après-vente.

### Brève description des dessins

### Brève description des figures

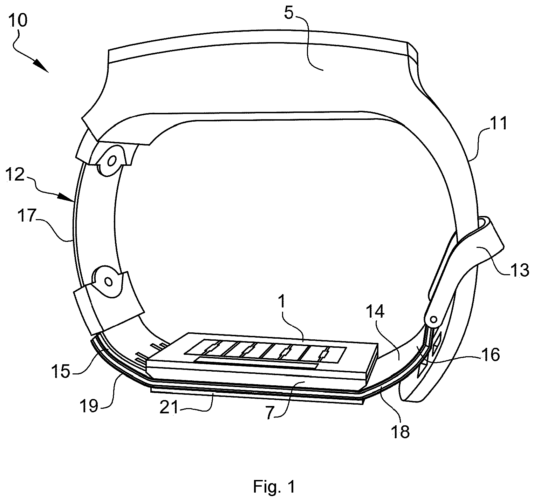

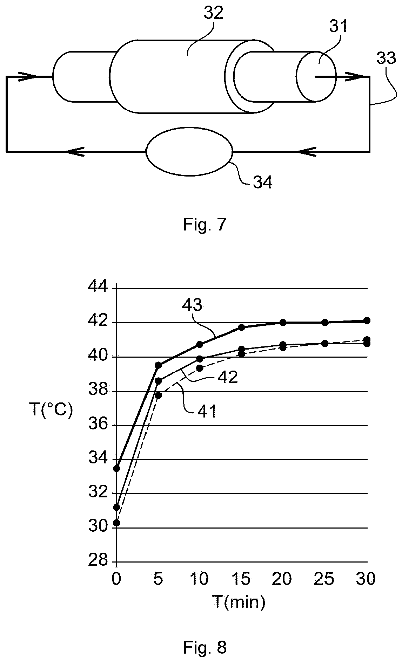

Nous allons maintenant présenter un mode de réalisation de l'invention à titre d'exemple en se référant aux dessins annexés dans lesquels :
est une vue en perspective d'un bracelet selon un premier mode de l'invention et partiellement transparent ;
est une vue partiellement éclatée du bracelet de la figure 1 ;
est une vue de côté et en coupe transversale du bracelet de la figure 1 ;
est une vue de côté et sans transparence du bracelet de la figure 1 ;
est une vue en perspective d'un brin et de certaines couches du bracelet de la figure 1 ;
est une vue similaire du même brin et d'une couche supplémentaire, illustrés de manière éclatée ;
est un schéma d'un modèle de bras visant à reproduire les conditions d'échauffement du bracelet de la figure 1 ;
est un graphique illustrant des mesures de température sur le modèle de la figure 7 et sur le bracelet de la figure 1 ; et
est un graphique illustrant d'autres mesures de température sur les mêmes éléments.

### Description des modes de réalisation

### Description détaillée

Un bracelet 10 selon un mode de réalisation de l'invention est illustré aux figures 1 à 4. Il s'agit d'un bracelet visant à traiter la douleur chez un patient le portant au poignet. Par l'émission d'ondes millimétriques, ce bracelet peut également servir à traiter le stress ou tout simplement à engendrer une sensation de bien-être chez ce patient.

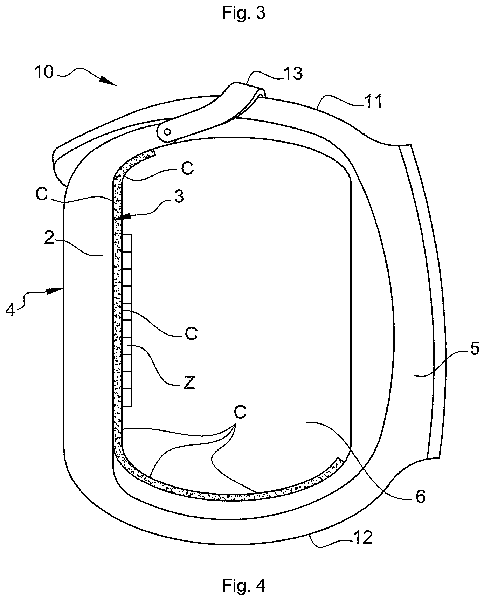

Le bracelet 10 permet l'envoi d'ondes à la surface de la peau, en particulier dans la zone référencée Z à la figure 4 et qui correspond à la partie inférieure du poignet 6, ici coupé transversalement, du patient. Fixé à la manière d'une montre autour du poignet 6, le bracelet 10 peut ainsi être porté sans inconvénient particulier par le patient et permettre son traitement sans inconfort. La zone Z correspond à une portion du corps humain particulièrement innervée. L'étude « Radzievsky AA, Rojavin MA, Cowan A, Alekseev SI, Ziskin MC. Hypoalgesic effect of millimeter waves in mice: Dependence on the site of exposure. Life sciences. 2000;66(21):2101-11 » a démontré l'effet thérapeutique avantageux de l'envoi d'ondes millimétriques dans de telles zones. Le port du bracelet 10 pour permettre l'envoi d'ondes dans cette zone Z est donc particulièrement intéressant.

On va maintenant décrire la structure du bracelet 10.

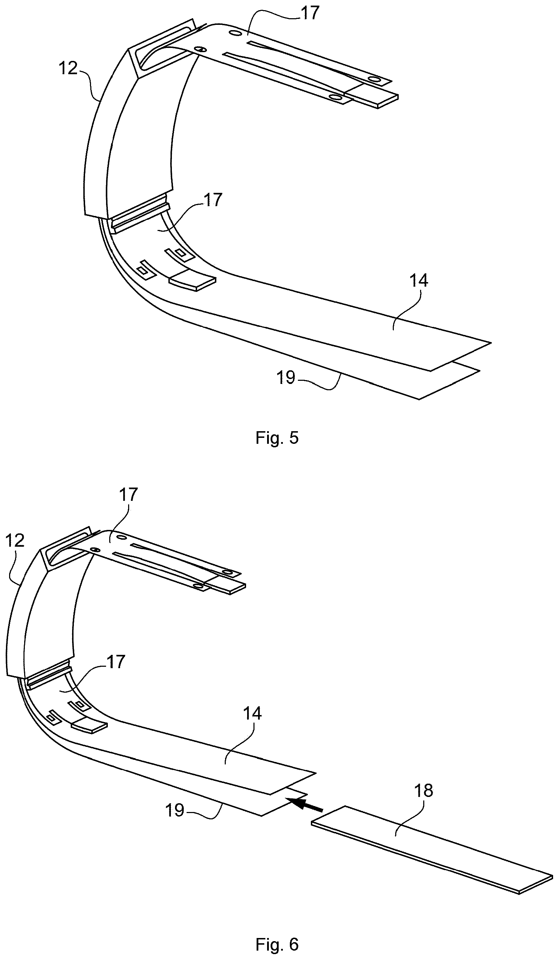

En référence aux figures 4 et 5, Le bracelet 10 présente un boitier rigide 2 destiné à être plaqué contre la partie inférieure Z du poignet humain 6 lorsque le bracelet 10 est fermé autour du poignet 6. Par rigide, on veut signifier que les surfaces du boitier ne sont pas aisément déformables par la main humaine, de la même manière qu'un boitier de montre par exemple. Ce boitier rigide 2 présente une paroi interne rigide 3 en polycarbonate destinée à être plaquée contre la partie inférieure Z du poignet humain. Le polycarbonate de cette paroi 3 permet de laisser passer les ondes émises par le module d'émission d'ondes 1 décrit plus bas. Alternativement, il peut s'agir d'un autre matériau transparent aux ondes millimétriques tel que du silicone. Le boitier 2 présente également, en face, une paroi externe rigide 4 en matière plastique, destinée à être en contact avec l'air ambiant. Ce boitier 2 comprend des éléments qui seront décrits plus bas.

Le bracelet 10 présente également deux brins 11 et 12 s'étendant respectivement de chaque côté du boitier 2 et depuis ce boitier. Ces deux brins 11 et 12 comprennent du silicone et forment des parties souples du bracelet, s'adaptant à la forme du poignet 6 du patient comme le font les brins d'une montre classique. Le brin 11 peut lui-même être subdivisé en deux sous-brins agencés pour former une boucle 13 permettant de fermer et d'ouvrir le bracelet. Cet agencement de fermeture et la boucle 13 ne feront pas l'objet de détails dans cette demande, et peuvent être de tout type. Les deux brins 11 et 12 s'étendent depuis le boitier 2 vers un autre boitier rigide 5, destiné lui à être placé sur la partie supérieure du poignet 6, comme l'est généralement le boitier (comprenant le cadran) d'une montre par exemple.

Ce boitier 5 s'étend donc en face du boitier 2, chacun étant plaqué contre deux parties opposées du poignet du patient, ces deux boîtiers rigides 2 et 5 étant donc reliés par des brins 11 et 12 souples. Ce boitier 5 comprend une interface permettant au patient ou à un médecin de paramétrer l'envoi d'émission d'ondes. Il contient également une batterie, voire d'autres éléments électroniques qui ne seront pas décrits en détails. L'électronique de ce boitier 5 permet de contrôler et d'alimenter le module d'émission d'ondes 1 décrit plus bas.

On va maintenant s'intéresser à l'émission d'ondes et à ses conséquences sur la chaleur émise.

Pour envoyer des ondes à la surface de la peau, le boitier rigide 2 enferme un module d'émission d'ondes 1, placé contre la paroi interne 3 du boitier 2 qui laisse passer ces ondes. Pour que l'émission d'ondes ait un effet médical, il est nécessaire que la densité surfacique de puissance des ondes émises soit d'au moins 0.5 milliwatts par centimètre carré de surface de peau irradiée. Concernant la fréquence de ces ondes, elle doit être inférieure à 300 gigaherz pour que le traitement soit efficace. On qualifie ces ondes de « millimétriques ». A ce sujet, l'étude Radzievsky AA, Gordiienko OV, Alekseev S, Szabo I, Cowan A, Ziskin MC : « Electromagnetic millimeter waves for pain therapy. Evid Based Complement Alternat Med » tend à montrer que l'effet optimal d'un traitement par ondes millimétriques est obtenu avec une fréquence autour des 61,25 GHz et une densité surfacique de puissance d'environ 13 mW/cm². Illustré aux figures 1 à 3, ce module 1 présente un circuit intégré en silicium dans un boitier de type BGA (« Ball Grid Array »), comprenant des billes (dites « bump »), le circuit étant soudé sur un substrat « HF » en PTFE (Polytétrafluoroéthylène) RO3003. Ce circuit imprimé, comprenant des agencements entre des ASIC et des antennes surfaciques ainsi qu'un circuit d'alimentation, permet l'envoi des ondes à destination de la peau, avec la densité surfacique et la fréquence souhaitées, et ce pendant la durée souhaitée. La surface de ce module 1 est en l'espèce de 20*37 millimètres, et la surface irradiée par les ondes est de 2.5 centimètres carrés de peau. Bien entendu, l'invention n'est pas limitée à un module 1 particulier. Par exemple, les dimensions et les matériaux pourraient être différents, de même que les composants électroniques.

Le traitement par ondes n'est pas continu. Il peut par exemple durer quelques minutes, être stoppé, puis redémarrer. Dans un mode de mise en oeuvre d'un traitement par envoi d'ondes, les ondes peuvent être émises pendant trente minutes de manière continue et de façon à générer une puissance rayonnée absorbée par la peau d'environ 35 milliWatts. Pour une telle puissance absorbée, il est nécessaire de fournir 1.25 watts au module 1. Or, le rendement de la conversion énergétique entre le courant reçu et le rayonnement électromagnétique, conversion réalisée par les ASIC du module 1, est faible. Les pertes thermiques sont donc importantes. En outre, l'élévation de la température peut être brutale.

Cette élévation de température a plusieurs inconvénients. D'une part, le rendement est d'autant plus élevé que la température du module 1 est faible. Il est donc nécessaire d'abaisser la température du module 1. D'autre part, la chaleur est transmise à la peau, notamment la portion Z du poignet 6, du patient. Or, il est nécessaire que l'élévation de température ne soit pas inconfortable pour le patient. Le ressenti du patient peut dépendre de nombreux facteurs - son activité du moment, la taille de la zone chauffante, la vitesse de la montée en température - mais on considère en général qu'une température maximale acceptable est de 43°C. C'est d'ailleurs également la température maximale fixée par la norme EN 60601-1, qui stipule que, pour une durée de contact supérieure à dix minutes entre un dispositif médical et une peau, la température de la partie appliquée sur la peau ne doit pas dépasser 43°C. Pour toutes ces raisons, l'invention prévoit un système de gestion de la dissipation thermique au sein du bracelet 10 qui vise à dissiper 1.25 watts de puissance thermique pendant trente minutes de manière à ce que la température la plus élevée en un point de la peau du patient ne dépasse pas 43°C.

On va maintenant décrire la structure du système de gestion de la dissipation thermique présent au sein du bracelet 10.

Tout d'abord, le bracelet 10 utilise la peau du patient comme vecteur de dissipation thermique. Des simulations ont en effet démontré que, malgré une faible superficie de contact, 25% de la chaleur est absorbée par la peau. Cela s'explique notamment par la forte perfusion de la peau à l'intérieur du poignet 6. Il est donc pertinent d'optimiser la surface de contact de la peau avec la source de chaleur. Cela se fait d'une part en plaquant au mieux la partie inférieure du poignet 6 contre le module d'émission d'ondes 1, d'autre part en dissipant la chaleur le long du bracelet 10 et en plaquant ce bracelet au mieux le long de la peau. Cela est réalisé en partie grâce aux brins souples 11 et 12, qui s'adaptent à la forme et aux dimensions du poignet 6 du patient. Mais il est d'abord nécessaire de pouvoir dissiper la chaleur au sein du bracelet pour qu'elle soit ensuite absorbée par une plus grande surface de peau.

C'est notamment à cet effet que le bracelet 10 comprend plusieurs couches de matériau visant à dissiper la chaleur émise par le module 1 le long du brin 12. Cette dissipation permet également, en soi, de diminuer l'élévation de la température en répartissant la chaleur le plus loin possible du module 1. C'est cet empilement de couches, que l'on va décrire maintenant, qui permet de dissiper la chaleur émise le long du brin 12.

Au préalable, notons que le brin 12 présente en son sein une nappe 17, dite « flex », illustrée aux figures 5 et 6, qui consiste à connecter l'électronique du boitier 5 à celle du boitier 2, en particulier à celle formant le module 1 d'émission d'ondes.

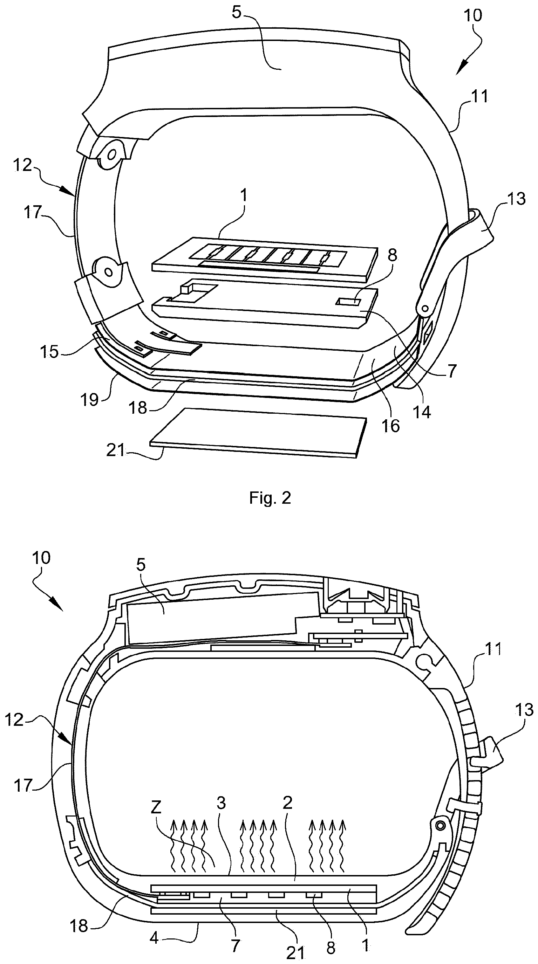

En référence aux figures 1 et 2, le boitier 2 comprend un pad thermique 7, dit « gap pad », de forme générale rectangulaire, qui permet de loger chaque ASIC du module 1 dans des cavités formées lors de l'assemblage - le gap pad étant mou et déformable - par exemple la cavité 8 illustrée aux figures 2 et 3. Le pad 7 agit ainsi comme logement des composants du circuit imprimé du module 1. Surtout, il permet de transférer au mieux la chaleur émise par ces composants à la couche 14 suivante, décrite après. En effet, ces composants ont différentes tailles, différentes positions, de sorte que le pad thermique 7 permet, grâce à ses cavités placées aux différents niveaux, de recouvrir uniformément l'ensemble du circuit et de transférer la chaleur émise par les composants où qu'ils soient. En l'espèce, le pad 7 illustré prend comme référence sur le marché « TGX-150-150 2.0-0 », de T-global Technology. Il est adapté en surface aux dimensions du module 1, et mesure 2 millimètres d'épaisseur. Ce pad 7 présente une conductivité thermique de 12 watts par mètre kelvin.

D'autres pads thermiques pourraient convenir. Il s'agit de trouver un compromis entre la plus forte conductivité thermique possible et le coût du pad. En termes de dimensions, l'objectif est d'assurer un bon contact avec les sources de chaleur du circuit, tout en ayant l'épaisseur la plus faible possible. L'épaisseur du pad doit donc être au moins équivalente à celle du composant le plus haut du circuit, avec une marge par exemple de 20 à 30%, tout en étant le moins épais possible.

En référence à la figure 5, en dessous du pad 7, le boitier 2 présente une première couche 14 de matériau conducteur, en l'espèce du graphite PGS (pour « Pyrolitic Graphite Sheet »), référencé EYGA091205PM chez Panasonic, qui présente une conductivité thermique de 1300 watts par mètre kelvin, s'étend sur une surface rectangulaire de 115 * 20 millimètres et présente une épaisseur de 50 micromètres. Cette couche permet d'évacuer la chaleur du module 1, transmise par le pad 7, vers l'extérieur du boitier 2. En effet, cette couche 14 s'étend depuis le boitier 2 vers et dans le brin 12, et vers la boucle 13, de chaque côté du boitier 2 et à l'extérieur de ce boitier, au sein de deux portions 15 et 16 courbées. Comme illustré aux figures 1 et 2, cette couche 14 est fixée par sa portion 15 d'une part à la nappe 17 du brin 12, et d'autre part, via sa portion 16, vers la boucle 13. Si le brin 11 est coupé en deux du fait de la boucle de fermeture 13, ce n'est pas le cas du brin 12. Ainsi, grâce à la fixation, par collage, de la portion 15 de la couche 14 sur la nappe 17, la chaleur émise par le module 1, transférée via le pad 7 à la couche 14, s'étend tout le long de la couche 14 au fort pouvoir conducteur, puis dans la nappe 17 le long du brin 12. C'est ainsi que l'on tire parti du pourtour du bracelet 10, en particulier d'une de ses parties souples, ici le brin 12, pour dissiper au maximum la chaleur émise par le module 1. Cette dissipation est illustrée schématiquement à la figure 4 où la chaleur est dessinée dans une zone C répartie au niveau du boitier 4, mais aussi tout le long du brin 12 et vers la boucle 13. Cette circulation de la chaleur, grâce à la conductivité thermique élevée de la couche 14, permet à la fois de diminuer l'élévation de température au niveau du module 1 et aussi de transférer la chaleur sur une plus grande surface de peau, de sorte que l'élévation de température ressentie par le patient est doublement amoindrie.

Alternativement, la couche conductrice 14 peut comprendre d'autres dimensions. Elle peut également présenter un matériau différent. Par exemple, elle peut comprendre du cuivre, qui présente une conductivité thermique d'environ 350 watts par mètre kelvin. Cependant, plus la conductivité est élevée, plus la dissipation est efficace et moins il est nécessaire que la couche soit épaisse, ce qui permet de gagner de la place et d'augmenter la flexibilité du bracelet. Il est donc nécessaire de trouver un compromis entre coût, conductivité la plus haute possible et épaisseur la plus faible possible. De manière générale, une gamme raisonnable d'épaisseur va de 0.01 à 1 millimètre pour permettre au brin 12 de rester flexible.

En référence aux figures 1 à 3, le boitier présente une deuxième couche 18, cette fois comprenant un matériau à changement de phase, aux dimensions surfaciques similaires à celles la couche 14, présentant une épaisseur de 0.740 millimètres et positionnée de manière adjacente à la couche conductrice 14. Le matériau ici implémenté est référencé « TSS EYGP0309RJAA » chez Panasonic et présente une enthalpie de changement de phase de l'ordre de 100 joules par gramme. Cette couche de matériau à changement de phase vise à reproduire, pour la température, le comportement d'un régime transitoire afin de limiter la brutalité de l'élévation de température. Ainsi, afin d'éviter d'atteindre l'équilibre thermique trop rapidement, on ajoute, via cette couche de matériau à changement de phase, de l'inertie au système afin de se retrouver dans un régime transitoire car l'inertie ainsi ajoutée permet de ne pas atteindre un équilibre dans un temps inférieur à la durée du traitement. Cette couche 18 de matériau à changement de phase joue donc le rôle de tampon thermique stockant les calories dissipées pendant la montée en température et qui lui sont transférées par la couche 14, afin de les restituer plus tard. Concrètement, elle permet d'absorber environ 450 joules, c'est-à-dire 20 % des 2250 joules (1.25 watts pendant 30 minutes) de chaleur émise pendant l'émission d'ondes. Cette couche de matériau à changement de phase 18 est prise en sandwich entre la couche conductrice 14 et une autre couche conductrice 19.

Bien entendu, la couche de matériau à changement de phase 18 pourrait présenter un autre matériau aux propriétés similaires. En général, ce type de matériau comprend des microparticules de paraffine ou d'autres matériaux aux propriétés similaires, avec notamment une température de fusion, et donc de changement de phase, autour de 35 à 40°C. Ses dimensions, en particulier son épaisseur, peuvent également différer. De manière générale, cette couche 18 de matériau à changement de phase doit posséder un volume et donc un stockage d'énergie possible suffisamment grand. En même temps, il faut que l'accès à ce matériau à changement de phase soit aisé : s'il est trop épais, la chaleur devra d'abord se diffuser à travers avant de faire changer de phase le matériau. C'est notamment pour cette raison que cette couche de matériau à changement de phase est entourée de deux couches conductrices, la couche 14 et la couche 19. Elles permettent d'optimiser la diffusion de chaleur en son sein. En cherchant un compromis raisonnable entre épaisseur et capacité à changer de phase rapidement, une épaisseur raisonnable de la couche de matériau à changement de phase devrait être située entre 1 et 5 millimètres.

La troisième couche 19 est une couche conductrice de chaleur similaire à la couche 14 par ses dimensions et identique par son matériau au fort pouvoir conducteur. Elle est placée contre la couche 18 de matériau à changement de phase, de sorte que les deux couches conductrices 14 et 19, similaires, entourent la couche de matériau à changement de phase 18. Cette couche 19 est ainsi elle-aussi fixée, au niveau de la nappe 17, à la couche de matériau à changement de phase 18.

Alternativement, son matériau peut être différent de celui de la couche 14. Là encore, l'objectif est de trouver un compromis entre une épaisseur raisonnable, une conductivité la plus élevée possible, et des coûts les plus faibles possibles. On peut par exemple prévoir que cette couche 18 soit en cuivre tandis que la couche 14 resterait en graphite.

Comme illustré notamment sur la figure 2, la dernière couche 21 se limite, en termes de dimensions, à la longueur du boitier rigide 2. Ainsi, contrairement aux couches 14, 18 et 19, la couche 21 ne s'étend pas en dehors du boitier rigide 2. Cette couche 21, placée contre la paroi rigide externe 4 du boitier 2, opposée à la paroi 3 où sont émises les ondes, est isolante thermiquement. Elle permet ainsi de limiter l'élévation de température sur le boitier 2 du côté 4 opposé à la paroi 3 d'où sont émises les ondes. Il s'agit donc de la couche interne au boitier 2 la plus proche de l'air ambiant de sorte que, en cas d'élévation de température de l'air ambiant, le point chaud correspondant à la présence des composants dissipant la chaleur est masqué de l'extérieur du boitier 2. Ainsi, la résistance thermique sur la surface du boitier 2 où est disposée cette couche isolante 21 est augmentée, forçant les calories à se propager plus loin le long du bracelet ou vers la peau. Par conséquent, la température ne dépasse pas la valeur maximale admissible au point le plus proche des composants.

Cette couche présente un matériau référencé « NASBIS EYGY0912QN4S » chez Panasonic et une épaisseur de 500 micromètres. Bien entendu, ce matériau et son épaisseur pourraient être différents. Il s'agit de trouver un compromis entre une épaisseur suffisamment faible et une capacité à isoler thermiquement le boitier suffisamment élevée.

On va maintenant résumer la structure du boitier 2.

Structurellement, le boitier 2 renferme donc un empilement comprenant, en partant de la paroi 3 accolée au poignet 6 humain, le module d'émissions d'onde 1 comprenant de l'électronique source de chaleur, le pad thermique 7, puis un empilement de trois couches 14, 18 et 19 dépassant du boitier 2 pour s'étendre dans le brin 12 et vers la boucle 13, et notamment fixées à la nappe électronique 17, les deux couches conductrices 14 et 19 entourant la couche de matériau à changement de phase 18. Enfin, sous la couche 19 se trouve une couche isolante 21 ne dépassant pas du boitier 2.

La chaleur émise par le module 1 est donc dissipée dans la couche 14, qui tire parti des portions souples du bracelet, en particulier du brin 12, pour étendre la surface de dissipation de la chaleur au maximum. De cette manière, la chaleur quitte la zone du module 1 et s'étale en partie sur le pourtour du poignet humain. On évite ainsi une concentration de l'élévation de température en un point. La chaleur est également transmise à la couche de matériau à changement de phase 18, qui permet d'absorber une partie de la puissance thermique, pour la restituer progressivement plus tard, après la fin de l'émission d'ondes. Cette couche 18, qui tire elle aussi parti des portions souples 11 et 12 du bracelet pour étendre son volume d'absorption et pour récupérer la chaleur dissipée par la couche 14, permet donc de capter une partie de la chaleur. Enfin, la chaleur restante est transmise à la couche 19 pour être dissipée sur la surface la plus grande possible, c'est-à-dire là aussi en s'étendant sur les portions souples 11 et 12 du bracelet. La couche isolante 21 permet, elle, de masquer le point chaud du boitier vis-à-vis de l'extérieur du boitier.

Le boitier 2 peut comprendre d'autres composants, par exemple un capteur de température ou plusieurs capteurs de température situés à différents endroits pour surveiller l'élévation de température de la peau du patient et/ou du module d'émission 1.

On va maintenant décrire les mécanismes de sécurité visant à faire face à une élévation trop importante de la température.

Les premières sécurités sont de type logiciel. En effet, le boitier 2 ou le boitier rigide 5, comprennent dans un support d'enregistrement un programme d'ordinateur visant à contrôler l'émission d'ondes. Alternativement ou en supplément, ce programme peut être détenu à distance. Dans tous les cas, un programme, contrôlé ou configuré par un utilisateur, automatise l'envoi d'ondes, en fonction d'un traitement médical prescrit ou d'une commande d'un l'utilisateur. On prévoit donc sur ce programme deux sécurités : la première prévoit de mettre en pause l'émission d'ondes si la température de la peau, température déterminée par un capteur prévu à cet effet et située sur le bracelet dans le boitier rigide 2, vient à dépasser 43°C. Une fois que la température mesurée redevient inférieure à 41 °C, l'émission d'ondes peut reprendre. On assure donc que la température de la peau ne dépasse pas 43°C, soit la norme en vigueur. Une deuxième sécurité logicielle, c'est-à-dire prévue dans le programme contrôlant l'émission d'ondes, prévoit de stopper l'émission d'ondes définitivement en cas de température dépassant 50°C. Cette sécurité est nécessaire en cas de dysfonctionnement de la sécurité précédente, ou en cas d'élévation importante subite de la température. Cette deuxième sécurité prévoit que l'émission ne peut être reprise que par une manipulation d'une personne agréée, par exemple provenant d'un service après-vente.

La troisième sécurité est de type matériel. Il s'agit de la dernière sécurité de secours, au cas où le programme d'ordinateur serait inopérant, par exemple supprimé ou amendé de manière hostile ou non intentionnelle. Ainsi, si le capteur de température prévu au niveau de la peau détecte une température au moins égale à 55°C, le module d'émission d'ondes est coupé directement au moyen d'un circuit électronique prévu à cet effet, que l'homme du métier sait déterminer. Il peut par exemple utiliser un composant référencé « TMP302ADRL » chez Texas Instrument et qui permet de couper l'alimentation des ASICs du module 1 en cas de température supérieure à une valeur prédéterminée, ici 55°C.

Ainsi, ces sécurités visent à éliminer tout risque d'élévation de température incontrôlée.

On va maintenant décrire le banc thermique qui a permis d'homologuer le bracelet et de démontrer que la température ne s'élevait pas au-dessus de celle prévue par la norme EN-60601-1, validant ainsi l'efficacité du système de gestion de la dissipation thermique décrit précédemment.

La norme EN-60601-1 définit, au sein de son paragraphe 11.1.1, en résumé, qu'une partie d'un dispositif médical en contact avec la peau ne peut avoir une température supérieure à 43°C à l'endroit le plus défavorable pendant plus de 10 minutes. C'est cette norme que vise à respecter le bracelet, étant donné que l'émission d'ondes peut durer plus de 10 minutes, en particulier 30 minutes. Le paragraphe 11.1.3 de la norme définit comment doit être testé l'appareil pour mesurer l'échauffement thermique.

La figure 7 illustre le modèle visant à reproduire les propriétés thermiques de la peau humaine pour tester le bracelet. Il comprend donc un conduit 31 reproduisant un bras, un gel d'agarose à 2% 32 simulant la peau du bras formant un manchon placé sur le conduit, manchon sur lequel est placé le bracelet 10. Ce gel est maintenu à une température similaire à celle de la peau humaine grâce à la circulation d'un fluide 33 à l'intérieur du conduit. Ce fluide reproduit également la circulation sanguine, laquelle permet d'évacuer une partie des calories issues de l'échauffement du bracelet 10. L'organe 34 est un dispositif permettant de maintenir à une température souhaitée le fluide 33, la température étant celle permettant d'obtenir une température de gel 32 similaire à celle de la peau.

Dans un premier temps, on a comparé des mesures de température sur dix participants humains et sur le bracelet 10, afin d'étalonner la température et le débit du fluide 33, en les faisant varier. Une fois les mêmes valeurs obtenues entre les températures mesurées sur le modèle et celles mesurées sur les humains, la température du fluide correspondante et son débit ont été déterminées.

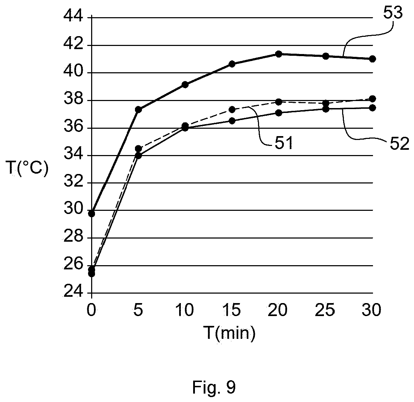

On a alors pu effectuer des mesures sur le modèle reproduisant les conditions du port d'un bracelet sur un humain. Les résultats sont illustrés aux figures 8 et 9. La figure 8 illustre les températures de la peau en contact avec le boitier 2, avec trois courbes : la courbe 41 est la mesure de la température moyenne sur 10 participants humains, pendant 30 minutes d'émission, avec une température ambiante de 25°C. La courbe 42 est la mesure réalisée sur le modèle de la figure 7, toujours sous 25°C. On remarque que ces courbes sont très proches, le modèle ayant même tendance à surestimer la température de la peau. La comparaison entre les courbes 41 et 42 a permis de valider la pertinence du modèle, lequel peut être reproduit en laboratoire aisément. Enfin, la courbe 43 représente la température prise sur le modèle, avec 30°C de température ambiante. Cette température de 30°C correspond à la température ambiante au-dessus de laquelle la notice d'utilisation recommandera de ne pas utiliser le bracelet 10. On remarque que toutes les courbes ne dépassent jamais la température de 43°C. Sous 30°C, la valeur maximale atteinte par le modèle est de 42°C et se stabilise à cette température à partir de 20 minutes. Pour 25°C, les deux courbes 41 et 42 montrent que la température maximale atteinte au niveau de la peau est inférieure à 41°C. La figure 9 illustre trois courbes 51, 52 et 53 correspondant à la température prise du côté de la paroi 4 du boitier, sous 25°C sur un humain (en moyenne sur 10 participants) pour la courbe 51, sur le modèle de la figure 7 pour la courbe 52 et sous 30°C pour le même modèle sur la courbe 53. On remarque qu'aucune température ne dépasse 43°C, ni même 42°C.

Ces tests ont permis de fabriquer un modèle de test adapté aux exigences de la norme, et de démontrer que dans le cas le plus défavorable - une puissance thermique de 1.25 watts pendant 30 minutes - à l'endroit le plus critique - entre le module et la peau - la température ne dépasse pas 43°C. Cette limitation de la température est rendue possible par le système de gestion de la dissipation thermique décrit plus haut.

L'invention n'est pas limitée aux modes de réalisation présentés et d'autres modes de réalisation apparaîtront clairement à l'homme du métier. Il est notamment possible d'utiliser le système de gestion de la dissipation thermique dans un autre type de bracelet, par exemple dans une montre connectée munie d'une ou plusieurs applications et qui n'aurait pas vocation à émettre des ondes. Le bracelet peut par exemple ne présenter qu'un seul boitier rigide. De plus, ce système pourrait être adapté à un autre type de dispositif tel qu'un smartphone où il gérerait la dissipation thermique par exemple de la carte mère du smartphone. Cela pourrait nécessiter l'adaptation des dimensions des différentes couches, en particulier des couches conductrices pour tirer parti le plus possible de la surface disponible pour dissiper la chaleur.

Enfin, le boitier 2 pourrait ne pas être rigide. Il pourrait par exemple s'agir d'un boitier « semi-rigide », voire d'un boitier souple. Les modes de réalisations décrits précédemment valent en effet également pour un boitier non rigide ou non strictement rigide, puisque la stratégie de dissipation thermique ne dépend pas de la souplesse du boitier. L'invention est définie dans les revendications suivantes :

## Revendications

1. Dispositif (10) comprenant:
- au moins un boitier (2) comprenant au moins une source de chaleur (1), et
- au moins un brin (11, 12) formant une partie plus souple que le boitier (2),
**caractérisé en ce que** le brin (11, 12) comprend deux couches de matériau conductrices de chaleur (14, 19) et une couche de matériau à changement de phase (18) située entre les deux couches de matériau conductrices de chaleur.

2. Dispositif (10) selon la revendication précédente, le dispositif étant un bracelet.

3. Dispositif (10) selon l'une quelconque des revendications précédentes, dans lequel le boitier est rigide.

4. Dispositif (10) selon l'une quelconque des revendications précédentes, dans lequel une valeur de conductivité thermique d'au moins l'une des couches conductrices de chaleur (14, 19) est supérieure ou égale à 350 watts par mètre-kelvin, de préférence supérieure ou égale à 1300 watts par mètre-kelvin.

5. Dispositif (10) selon l'une quelconque des revendications précédentes, dans lequel une valeur d'épaisseur d'au moins l'une des couches conductrices de chaleur (14, 19) est située entre 0.01 et 1 millimètre.

6. Dispositif (10) selon l'une quelconque des revendications précédentes, dans lequel une valeur d'enthalpie de changement de phase de la couche de matériau à changement de phase (18) est d'environ 100 joules par gramme.

7. Dispositif (10) selon l'une quelconque des revendications précédentes, dans lequel les deux couches de matériau conductrices de chaleur (14, 19) sont collées à deux surfaces opposées de la couche de matériau à changement de phase (18).

8. Dispositif (10) selon l'une quelconque des revendications précédentes, comprenant en outre une nappe de connexion électronique (17) s'étendant depuis le boitier (2) et dans le brin (12), au moins l'une des couches de matériau conductrices de chaleur (14, 19) étant fixée à la nappe (17) dans le brin (12).

9. Dispositif (10) selon l'une quelconque des revendications précédentes, dans lequel au moins l'une des couches de matériau conductrices de chaleur (14, 19) comprend une feuille de graphite pyrolitique présentant une valeur d'épaisseur située entre 10 et 100 micromètres.

10. Dispositif (10) selon l'une quelconque des revendications précédentes, dans lequel la couche de matériau à changement de phase (18) présente une valeur d'épaisseur située entre 1 et 5 millimètres.

11. Dispositif (10) selon l'une quelconque des revendications précédentes, dans lequel, les trois couches (14, 18, 19) étant trois premières couches, le dispositif (10) comprend en outre une quatrième couche de matériau isolante thermique (21), située entre les trois couches (14, 18, 19) d'une part et une extrémité (4) du boitier (2) d'autre part.

12. Dispositif (10) selon la revendication précédente, dans lequel une valeur de conductivité de la couche de matériau isolante thermique (21) est située entre 0.018 et 0.026 watts par mètre kelvin, cette couche comportant de préférence une feuille thermo-isolante comprenant un aérogel de silice et des nanofibres, cette couche présentant de préférence une valeur d'épaisseur située entre 0.1 et 1 millimètre.

13. Dispositif (10) selon l'une quelconque des revendications précédentes, dans lequel la source de chaleur (1) est un module d'émission d'ondes (1) apte à émettre des ondes présentant une valeur de fréquence située entre 3 et 120 gigahertz et une valeur de densité surfacique de puissance supérieure ou égale à 0.5 milliwatts par centimètre carré.

14. Dispositif (10) selon l'une quelconque des revendications précédentes, dans lequel le boitier (2) comprend en outre un capteur de température apte à stopper une émission de chaleur de la source de chaleur (1) si une valeur de température du dispositif dépasse un seuil prédéterminé, par exemple si une valeur de température de la source de chaleur dépasse 55°C.

15. Dispositif (10) selon l'une quelconque des revendications précédentes, incluant en outre un support électronique comprenant sous forme enregistrée un programme d'ordinateur, le programme comportant des instructions de code aptes à activer et/ou désactiver une émission de chaleur de la source de chaleur en fonction d'une valeur de température du dispositif, par exemple apte à désactiver l'émission si la valeur est supérieure à 43°C et à activer l'émission si la valeur est inférieure à 41°C.

## Patentansprüche

1. Vorrichtung (10), umfassend
- wenigstens ein Gehäuse (2), das wenigstens eine Wärmequelle (1) umfasst, und
- wenigstens ein Band (11, 12), das einen Teil bildet, der flexibler ist als das Gehäuse (2),
**dadurch gekennzeichnet, dass** das Band (11, 12) Wärmeleitmaterialschichten (14, 19) und eine Phasenwechselmaterialschicht (18), die sich zwischen den zwei Wärmeleitmaterialschichten befindet, umfasst.

2. Vorrichtung (10) nach dem vorhergehenden Anspruch, wobei die Vorrichtung ein Armband ist.

3. Vorrichtung (10) nach einem der vorhergehenden Ansprüche, wobei das Gehäuse steif ist.

4. Vorrichtung (10) nach einem der vorhergehenden Ansprüche, wobei ein Wert der Wärmeleitfähigkeit wenigstens einer der Wärmeleitschichten (14, 19) größer oder gleich 350 Watt pro Meter und Kelvin ist, bevorzugt größer oder gleich 1300 Watt pro Meter und Kelvin.

5. Vorrichtung (10) nach einem der vorhergehenden Ansprüche, wobei ein Wert der Dicke wenigstens einer der Wärmeleitschichten (14, 19) zwischen 0,01 und 1 Millimeter liegt.

6. Vorrichtung (10) nach einem der vorhergehenden Ansprüche, wobei ein Wert der Phasenwechsel-Enthalpie der Phasenwechselmaterialschicht (18) etwa 100 Joule pro Gramm beträgt.

7. Vorrichtung (10) nach einem der vorhergehenden Ansprüche, wobei die zwei Wärmeleitmaterialschichten (14, 19) auf zwei gegenüberliegenden Flächen der Phasenwechselmaterialschicht (18) verklebt sind.

8. Vorrichtung (10) nach einem der vorhergehenden Ansprüche, umfassend ferner eine elektronische Verbindungslage (17), die sich von dem Gehäuse (2) aus und in das Band (12) erstreckt, wobei wenigstens eine der Wärmeleitmaterialschichten (14, 19) an der Lage (17) in dem Band (12) befestigt ist.

9. Vorrichtung (10) nach einem der vorhergehenden Ansprüche, wobei wenigstens eine der Wärmeleitmaterialschichten (14, 19) eine pyrolytische Graphitfolie umfasst, die einen Wert der Dicke aufweist, der zwischen 10 und 100 Mikrometer liegt.

10. Vorrichtung (10) nach einem der vorhergehenden Ansprüche, wobei die Phasenwechselmaterialschicht (18) einen Wert der Dicke aufweist, der zwischen 1 und 5 Millimeter liegt.

11. Vorrichtung (10) nach einem der vorhergehenden Ansprüche, wobei, wenn die drei Schichten (14, 18, 19) drei erste Schichten sind, die Vorrichtung (10) ferner eine vierte Wärmedämmmaterialschicht (21) umfasst, die sich zwischen den drei Schichten (14, 18, 19) einerseits und einem Ende (4) des Gehäuses (2) andererseits befindet.

12. Vorrichtung (10) nach dem vorhergehenden Anspruch, wobei ein Wert der Leitfähigkeit der Wärmedämmmaterialschicht (21) zwischen 0,018 und 0,026 Watt pro Meter und Kelvin liegt, wobei diese Schicht bevorzugt eine Wärmdämmfolie ist, die ein Aerogel aus Siliziumoxid und Nanofasern umfasst, wobei diese Schicht bevorzugt einen Wert der Dicke aufweist, der zwischen 0,1 und 1 Millimeter liegt.

13. Vorrichtung (10) nach einem der vorhergehenden Ansprüche, wobei die Wärmequelle (1) ein Modul zum Emittieren von Wellen (1) ist, das in der Lage ist, Wellen zu emittieren, die einen Wert der Frequenz, der zwischen 3 und 120 Gigahertz liegt, und einen Wert der Leistungsdichte größer oder gleich 0,5 Milliwatt pro Quadratzentimeter aufweisen.

14. Vorrichtung (10) nach einem der vorhergehenden Ansprüche, wobei das Gehäuse (2) ferner einen Temperatursensor umfasst, der in der Lage ist, eine Wärmeemission der Wärmequelle (1) zu stoppen, wenn ein Wert der Temperatur der Vorrichtung einen vorbestimmten Schwellenwert übersteigt, zum Beispiel, wenn ein Wert der Temperatur der Wärmequelle 55 °C übersteigt.

15. Vorrichtung (10) nach einem der vorhergehenden Ansprüche, ferner aufweisend einen elektronischen Träger, der in aufgezeichneter Form ein Computerprogramm umfasst, wobei das Programm Code-Anweisungen aufweist, die in der Lage sind, eine Wärmeemission der Wärmequelle in Abhängigkeit von einem Wert der Temperatur der Vorrichtung zu aktivieren und/oder zu deaktivieren, zum Beispiel in der Lage, die Emission zu deaktivieren, wenn der Wert größer ist als 43 °C, und die Emission zu aktivieren, wenn der Wert kleiner ist als 41 °C.

## Claims

1. Device (10) comprising:
- at least one housing (2) having at least one heat source (1), and
- at least one strand (11, 12) forming a more flexible part than the housing (2),
**characterized in that** the strand (11, 12) comprises two layers (14, 19) of heat-conducting material and a layer (18) of phase change material situated between the two layers of heat-conducting material.

2. Device (10) according to the preceding claim, the device being a bracelet.

3. Device (10) according to any one of the preceding claims, wherein the housing is rigid.

4. Device (10) according to any one of the preceding claims, wherein a thermal conductivity value of at least one of the heat-conducting layers (14, 19) is greater than or equal to 350 watts per metre-kelvin, preferably greater than or equal to 1300 watts per metre-kelvin.

5. Device (10) according to any one of the preceding claims, wherein the thickness of at least one of the heat-conducting layers (14, 19) is between 0.01 and 1 millimetre.

6. Device (10) according to any one of the preceding claims, wherein the phase change enthalpy of the layer (18) of phase change material is approximately 100 joules per gram.

7. Device (10) according to any one of the preceding claims, wherein the two layers (14, 19) of heat-conducting material are bonded to two opposite surfaces of the layer (18) of phase change material.

8. Device (10) according to any one of the preceding claims, further comprising an electronic connection flex (17) extending from the housing (2) and into the strand (12), at least one of the layers (14, 19) of heat-conducting material being attached to the flex (17) in the strand (12).

9. Device (10) according to any one of the preceding claims, wherein at least one of the layers (14, 19) of heat-conducting material comprises a sheet of pyrolytic graphite of thickness between 10 and 100 micrometres.

10. Device (10) according to any one of the preceding claims, wherein the thickness of the layer (18) of phase change material is between 1 and 5 millimetres.

11. Device (10) according to any one of the preceding claims, wherein, since the three layers (14, 18, 19) are first three layers, the device (10) further comprises a fourth layer (21) of heat-insulating material, situated between the three layers (14, 18, 19) firstly and an end (4) of the housing (2) secondly.

12. Device (10) according to the preceding claim, wherein the conductivity of the layer (21) of heat-insulating material is between 0.018 and 0.026 watts per metre-kelvin, this layer preferably comprising a heat-insulating sheet comprising a silica aerogel and nanofibres, the thickness of this layer preferably being between 0.1 and 1 millimetres.

13. Device (10) according to any one of the preceding claims, wherein the heat source (1) is a wave transmission module (1) adapted to transmit waves of frequency between 3 and 120 gigahertz and surface power density greater than or equal to 0.5 milliwatts per square centimetre.

14. Device (10) according to any one of the preceding claims, wherein the housing (2) further comprises a temperature sensor adapted to stop the emission of heat from the heat source (1) if the device temperature exceeds a predetermined threshold, for example if the temperature of the heat source exceeds 55°C.

15. Device (10) according to any one of the preceding claims, further including an electronic medium comprising a stored computer program, the program comprising code instructions adapted to activate and/or deactivate emission of heat from the heat source depending on the device temperature, for example adapted to deactivate emission if the temperature is greater than 43 °C and activate emission if the temperature is less than 41 °C.
